# EUROPEAN PATENT SPECIFICATION

(11) **EP 1 835 290 B1**
(45) Date of publication and mention of the grant of the patent: **04.05.2011**
(21) Application number: 06005531.6
(22) Date of filing: 17.03.2006
(51) Int. Cl.: G01N 33/94, G01N 33/74

(54) **Assay for catecholamines**
Katecholamin-Assay
Analyse pour catécholamines

(43) Date of publication of application: 19.09.2007
(73) Proprietor: Labor Diagnostika Nord GmbH & Co. KG, 48531 Nordhorn (DE)
(72) Inventor: Manz, Bernhard, Dr., 48527 Nordhorn (DE)
(74) Representative: Schnappauf, Georg

(56) References cited:
- DE-A1- 19 818 485
- WESTERMANN JÜRGEN ET AL: "Simple, rapid and sensitive determination of epinephrine and norepinephrine in urine and plasma by non-competitive enzyme immunoassay, compared with HPLC method." CLINICAL LABORATORY. 2002, vol. 48, no. 1-2, 2002, pages 61-71, XP002393227 ISSN: 1433-6510
- FAIRBANKS V F ET AL: "Measurement of glycosylated hemoglobin by affinity chromatography." MAYO CLINIC PROCEEDINGS. MAYO CLINIC. NOV 1983, vol. 58, no. 11, November 1983 (1983-11), pages 770-773, XP009070496 ISSN: 0025-6196
- ANONYMOUS: "CatCombi ELISA (RE59242)" IBL HAMBURG INSTRUCTION FOR USE, [Online] 27 February 2006 (2006-02-27), XP002393228 Retrieved from the Internet: URL:http://www.ibl-hamburg.com/fileadmin/u ser_upload/catalogue-files/IFU/RE59242_IFU _en_CatCombi%20ELISA_V4_2006_02.pdf>
- CANNIZZO C ET AL: "Boronic acid-functionalized nanoparticles: synthesis by microemulsion polymerization and application as a re-usable optical nanosensor for carbohydrates" POLYMER, ELSEVIER SCIENCE PUBLISHERS B.V, GB, vol. 46, no. 4, 7 February 2005 (2005-02-07), pages 1269-1276, XP004721923 ISSN: 0032-3861
- IKEDA K ET AL: "Determination of glycated albumin by enzyme-linked boronate immunoassay (ELBIA)." CLINICAL CHEMISTRY. FEB 1998, vol. 44, no. 2, February 1998 (1998-02), pages 256-263, XP002393229 ISSN: 0009-9147
- PIERCE BIOTECHNOLOGY INC.: 'Instructions: Immobilized boronic acid gel' March 2004,
- PETERSON K.P. ET AL: 'What is haemoglobin AI c? An analysis of glycated haemoglobins by electrospray ionisation mass spectrometry' CLINICAL CHEMISTRY vol. 44, no. 9, 1998, pages 1951 - 1958
- LI ET AL: 'Separation of neoglycoproteins with different degrees of glycosylation by boronate chromatography' vol. 54, no. 3/4, August 2001, page 213
- 'Affi-Gel 601 boronate affinity gel to separate ribonucleotides, ribonucleosides, sugars, catecholamines, and coenzymes.' BIO-RAD 10 December 1995,
- REINHARD WESEMAN: 'Radioimmunologische Bestimmung von Catecholaminen und deren Metabolite' DIPLOMARBEIT FACHHOCHSCHULE HAMBURG FACHBEREICH CHEMIINGENIEURWESEN July 1991,
- MANZ B. ET AL: 'Radioimmunologische Bestimmung des Adrenalins/Noradrenalins sowie des Metanephrins in Urin' GIT LABOR-MEDIZIN vol. 5, no. 90, 1990, pages 245 - 248

## Description

### TECHNICAL FIELD OF THE INVENTION

The present invention relates to the analysis of catecholamines. In particular, the invention relates to affinity extraction and detection and/or quantification of catecholamines.

### BACKGROUND OF THE INVENTION

Catecholamines like norepinephrine, epinephrine and dopamine play an important role as neurotransmission substances or hormones. In order to study the functions and activities of sympathetic nerves, it is important to determine quantitative changes of catecholamines in a living body sample such as urine or plasma. Additionally, abnormal catecholamine concentrations are associated with a variety of diseases, for example hypertonia, pheochromocytoma, sympathetic neuroblastoma, degenerative cardiac diseases, schizophrenia, and alternating psychosis (Manz B. et al. (1990) GIT Labor-Magazin 5/90. 245-254).

Catecholamines are very unstable and readily undergo oxidation, in particular under basic conditions. Furthermore, the concentration of catecholamines especially in plasma samples is quite low. Thus, there is a need for quick and sensitive determination methods for catecholamines.

To date, the following methods are available for the determination of catecholamines: fluorometric assays, radio enzymatic assays (REA), high-performance liquid chromatography (HPLC) in combination with different detection techniques, gas chromatography with mass spectrometric detection (GC-MS), radio immunoassays (RIA) and enzyme immunoassays (EIA) (Manz B. et al. (1990) GIT Labor-Magazin 5/90, 245-254; Wolthers B.G. et al. (1997) Clinical Chemistry 43, 114-120). However, the fluorometric and radio enzymatic assays lack accuracy and reproducibility and the fluorometric assays do not differ between the different catecholamines. HPLC and GC-MS are time consuming, need complex technical equipment and, therefore, are cost-intensive. Thus, the most suitable methods for determining catecholamines in view of reliability, cost, accuracy, reproducibility and rapidness are the immunoassays.

In currently available EIA- or RIA-based assays for determining catecholamines, the catecholamines are first extracted out of the sample, then derivatized for antibody recognition and to enhance their stability, and thereafter detected using antibodies specific for catecholamines. Thereby, the most commonly used extraction method is binding of the catecholamines via their cis-diol structure to a boronate affinity gel, see e.g. DE 198 18 485; Cannizzo C. et al. (2005) Polymer 46, 1269-1276. The subsequent modification to stabilize the catecholamines is done using the enzyme catechol-O-methyl-transferase (COMT) which O-methylates the 3-hydroxyl group of the catechol moiety, see Westemann J. et al. (2002) Clinical Laboratory 48, 61-71; IBL Hamburg, "Instructions for Use: CatCombi ELISA", version 4 / 2006-02-27. Thus, for the COMT to access the 3-hydroxyl group of the catechol moiety, the catecholamines have to be released from the affinity gel which is achieved by adjusting the pH to acidic conditions. However, COMT is not active under acidic conditions. Therefore, in order to allow the reaction catalyzed by COMT to take place and prevent rebinding of the catecholamines released from the boronate affinity gel, the released catecholamines have to be separated from the boronate affinity gel by transferring them into another reaction vessel.

This transfer of the sample, however, creates an additional source of error and complicates the assay.

A known principle for reducing the transfer steps in methods for analyzing catecholamines by EIA is to perform the enzymatic O-methylation and the subsequent EIA in one reaction vessel. However, a drawback of this method is the fact that derivatization of the catecholamines and binding to the specific antibodies proceed in a parallel manner. Since only derivatized catecholamines are detected by the used antibodies this results in lowered precision and kinetics of the EIA and thus, in a prolonged assay time. Furthermore, this method cannot be performed in standard automated EIA analyzers due to the preceding enzymatic reaction taking place in the same reaction vessel.

Immobilized boronic acid gels containing m-aminophenylboronic acid are used as affinity support for purification of nucleotides and other small molecular weight compounds containing cis-diol groups and for measuring glycosylated hemoglobin (see Pierce Biotechnology, Inc., "Instructions: Immobilized Boronic Acid Gel", 3/2004; Fairbanks V.F. et al. (1983) Mayo Clinic Proceedings 58, 770-773; Peterson K.P. et al. (1998) Clinical Chemistry 44, 1951-1958).

Thus, there is a need for a method for analyzing catecholamines wherein no transfer step during the extraction and derivatization procedures is necessary. Such a method would eliminate a source of error and enable the separation of the extraction and derivatization procedures from the EIA, thereby allowing the use of standard automated EIA analyzers.

### SUMMARY OF THE INVENTION

The present invention relates to a method for analysis of catecholamines which does not require the step of transferring the sample in a different reaction vessel to avoid rebinding of catecholamines to the cis-diol specific affinity medium used for extraction of the catecholamines from the sample. In particular, according to the present invention, the released catecholamines have not to be separated from the cis-diol specific affinity medium prior to adjusting the pH to non-acidic conditions which may be required, for example, for subsequent enzymatic reactions, in particular that catalyzed by COMT.

Accordingly, the present invention relates to a method for analyzing at least one catecholamine comprising the steps of (a) binding the at least one catecholamine to a cis-diol specific affinity medium, (b) adding a substance that blocks the binding of catecholamines to the cis-diol specific affinity medium by competitive binding to the cis-diol specific affinity medium;
(c) releasing the at least one catecholamine bound to the cis-diol specific affinity medium from the cis-diol specific affinity medium; and
(d) subjecting the at least one catecholamine to at least one reaction which requires non-acidic conditions;
wherein step (b) and step (c) are performed simultaneously, or step (b) is performed prior to or following step (c), and wherein step (a), step (b), step (c) and step (d) are all done within the same reaction vessel.

Preferably, the at least one catecholamine comprises a naturally occurring and/or a synthetic catecholamine. More preferably, the naturally occurring catecholamine is selected from the group consisting of adrenalin, noradrenaline, dopamine, epinephrine, norepinephrine and mixtures thereof.

In one embodiment of the method of the present invention, the at least one catecholamine is contained in a biological sample or a fraction thereof. Preferably, the biological sample is a urine sample or a plasma sample.

In a preferred embodiment of the method of the present invention, the cis-diol specific affinity medium is a boronate affinity medium, preferably comprising amino boronic acid groups, more preferably m-aminophenyl boronic acid groups.

In one embodiment of the method of the present invention, the cis-diol specific affinity medium comprises cis-diol specific groups linked to a reaction vessel which may be a member selected from the group consisting of macro- and microtiter plates, pin plates, multi-well plates, cell and tissue culture plates and wells, test tubes and flasks. Preferably, the reaction vessel is in a pin plate format wherein the catecholamine or the antibody specific for a catecholamine specific antibody is immobilized on one or more pins of the pin plate. In particular, the catecholamine immobilized to the reaction vessel is preferably acylated and/or O-alkylated and the antibody immobilized to the reaction vessel is preferably specific for an antibody specific for an acylated and/or O-alkylated catecholamine.

Preferably, step (a) takes place at about neutral to basic pH, more preferably at a pH of about 6 or higher, even more preferably at a pH from about 7 to about 10.

In one embodiment of the method of the present invention, the substance added in step (b) contains at least one cis-diol structure, and more preferably is selected from the group consisting of 1,2-dihydroxyalkanes, polyphenols, 2-hydroxy acids, ascorbic acid, carbohydrates, derivatives thereof and mixtures thereof. The 1,2-dihydroxyalkane may be ethanediol, 1,2-propanediol, or an alkanediol comprising vicinal hydroxyl groups. The polyphenol may be a catechol, anthocyane, gallic acid, or tannin. The 2-hydroxyacid may be lactic acid, tartaric acid, or citric acid. The carbohydrate may be a monosaccharide, a disaccharide, an oligosaccharide or a polysaccharide. The carbohydrate derivative may be a nucleoside, a nucleotide, an oligo- or polynucleotide such as RNA, or a glycoprotein. Preferably, the substance is a saccharide such as sorbitol or fructose, ascorbic acid or a nucleotide.

The method of the present invention comprises the step of releasing the at least one catecholamine bound to the cis-diol specific affinity medium from the cis-diol specific affinity medium. Step (b) and the step of releasing the at least one catecholamine may be performed simultaneously or step (b) may be performed prior to or following the step of releasing the at least one catecholamine. Preferably, the at least one catecholamine is released from the cis-diol specific affinity medium by addition of an acid including organic acids, inorganic acids and mixtures thereof. Organic acids include acetic acid, propionic acid, pyruvic acid, malonic acid, succinic acid, maleic acid, fumaric acid, benzoic acid, cinnamic acid, methane sulfonic acid, ethane sulfonic acid, p-toluene sulfonic acid, salicylic acid, glycolic acid, lactic acid, oxalic acid, malic acid, citric acid, and mandelic acid.

Inorganic acids include sulfuric acid, nitric acid, phosphoric acid, perchloric acid, and hydrohalic acids such as hydrochloric acid, hydrobromic acid and hydroiodic acid. Preferably, the acid is hydrochloric acid or perchloric acid. Furthermore, in the step of releasing the at least one catecholamine from the cis-diol specific affinity medium the pH is preferably about 3 or lower, more preferably from about 1 to about 2.

In one embodiment, the method of the present invention further comprises a rinsing step following step (a) and prior to the step of releasing the at least one catecholamine from the cis-diol specific affinity medium.

In one embodiment of the method of the present invention, the substance added in step (b) is added to a final concentration that (i) is sufficient to block the binding of at least 90%, preferably at least 95%, more preferably at least 98% and most preferably at least 99% of the at least one catecholamine released from the cis-diol specific affinity medium to the cis-diol specific affinity medium, and/or (ii) equals or exceeds the binding capacity of the used cis-diol specific affinity medium. Preferably, the ratio between the final concentration of the substance added in step (b) and the binding capacity of the used cis-diol specific affinity medium is in the range of from about 1:1 to about 5000:1.

Furthermore, in the method of the present invention, following step (b) and the step of releasing the at least one catecholamine from the cis-diol specific affinity medium the at least one catecholamine is subjected to at least one reaction which requires non-acidic conditions, preferably an enzymatic reaction. Preferably, the reaction is an O-alkylation, more preferred an O-methylation. Most preferably, the O-methylation is effected by means of the enzyme catechol-3-O-methyl-transferase in its soluble or membrane bound form. Furthermore, the non-acidic conditions may include a pH of about 6 or higher, preferably of from about 6 to about 9.

In one embodiment, the method of the present invention further comprises the step of acylating the at least one catecholamine. The acylation may be effected by Bolton-Hunter reagent. Preferably, the at least one catecholamine is acylated while bound to the cis-diol specific affinity medium. Furthermore, a rinsing step following acylation of the at least one catecholamine and prior to the step of releasing the at least one catecholamine from the cis-diol specific affinity medium may be included in the method of the present invention.

In one embodiment, the method of the present invention further comprises the step of detecting and/or quantifying the at least one catecholamine, preferably using an antibody specific for a catecholamine or modified catecholamine. Preferably, the detection and/or quantification comprises an enzyme or radio immunoassay.

In one embodiment of the method of the present invention, the substance added in step (b) does not interfere with the following reactions or with a subsequent immunoassay.

In one embodiment of the method of the present invention, the step of detection and/or quantification is also done within the same reaction vessel.

### DEFINITIONS

According to the present invention, the term "catecholamine" refers to naturally occurring catecholamines such as adrenalin, noradrenaline, dopamine, epinephrine and norepinephrine as well as synthetic catecholamines, i.e. catecholamines not found in nature, such as benserazide, carbidopa, dobutamine, dopexamine, isoprenaline and alpha-methyldopa, mixtures thereof and derivatives thereof. The general formula of naturally occurring catecholamines is as follows: 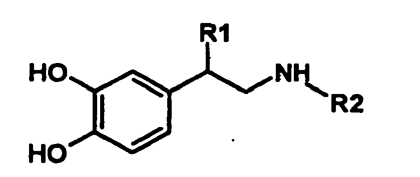 wherein R1 is OH for adrenalin and noradrenalin, and H for dopamine, and R2 is H for noradrenalin and dopamine, and CH₃ for adrenalin. Epinephrine and norepinephrine are the physiologically active R-enantiomeres (according to the Cahn-Ingold-Prelog system) of adrenaline and noradrenaline, respectively.

It is noted that synthetic catecholamines may comprise further substituents in addition to those indicated in the above formula.

Catecholamines determined by the method of the present invention may be contained in a sample derived from a natural source, i.e. a biological sample, in particular bodily fluids.

The term "biological sample" according to the present invention comprises any material which may be used for testing in the methods according to the present invention. The sample may be derived from any source and may include components of different sources. As used herein, a "biological sample" is any sample obtained from or present in a biological system such as humans and animals.

The biological samples can be obtained by methods known in the art such as venous puncture (blood, serum, plasma), directly collecting urine or collecting urine by use of a catheter. Preferably, the biological sample is a blood, serum, plasma, or urine sample, preferably obtained from a healthy animal or human or from an animal or human afflicted with a disease or other condition.

"Fraction of a biological sample" means for example a fraction obtained from a biological sample by purifying, fractionating, separating etc. the biological sample with methods such as chromatography, ultrafiltration, precipitation techniques, etc.

Terms such as "analyzing catecholamines" or "analyzing at least one catecholamine" relate to methods of measuring, collecting and analyzing data about catecholamines such as their relative or absolute concentration, their presence or absence, etc. and include, in particular, the relative or absolute quantification of catecholamines.

The term "analyzing at least one catecholamine" according to the invention refers to an analysis of all or only a fraction of the catecholamines contained in a sample, in particular 1, 2, up to 4, up to 6, up to 8 or up to 10 different catecholamines. This can be achieved, for example, by using antibodies being either specific for a particular catecholamine or derivative thereof or a particular group of catecholamines or derivatives thereof or all catecholamines or derivatives thereof and/or using a mixture of antibodies having different specificities.

Terms such as "analyzing catecholamines" or "analyzing at least one catecholamine" according to the invention also includes situations wherein no catecholamines are detected or the amount of said catecholamines is below the detection limit.

According to the invention, a "reference sample" may be used to correlate and compare the results obtained from a test sample. The composition of a "reference sample" is usually similar to a test sample but differs from the test sample in certain variables, e.g. the test sample may be derived from an individual suspected of being afflicted with a disease while the reference sample is obtained from a healthy individual and/or an individual known to be afflicted with said disease.

According to the present invention, the term "derivative" in connection with a particular compound or groups of compounds refers to a modified form of said compound(s). In one embodiment, a derivative of a compound retains the function or activity of the parent compound such as the ability to bind to cis-diol specific groups. In another embodiment the derivatization of a compound creates a property which was not present in a parent compound or modifies, preferably enhances a property of a parent compound such as the stability of catecholamines or capability of catecholamines to interact with antibodies. Preferably, a derivative of a compound comprises one or more substituents at one or more positions of the parent compound. Derivative of a compound also includes substances wherein said compound is only a minor constituent of the substance. For example, according to the present invention a RNA molecule may be considered as a derivative of a ribonucleotide. Particular examples of catecholamine derivatives or modified catecholamines are catecholamines modified by alkylation, preferably O-alkylation, more preferably O-methylation, and/or modified by acylation.

According to the present invention a cis-diol specific affinity medium may be used to extract catecholamines from a sample. As used herein, a "cis-diol specific affinity medium" comprises a support (matrix) which exhibits chemical structures (cis-diol specific groups) that specifically bind to substances comprising a cis-diol structure. A cis-diol structure comprises two hydroxyl groups, each bound to one of two adjacent carbon atoms, respectively.

The matrix of the cis-diol specific affinity medium may be a polymer, e.g. polyacrylamide, which preferably forms a gel-like structure, or the matrix may be the wall of a reaction vessel such as macro- and microtiter plates, pin plates, multi-well plates, cell and tissue culture plates and wells, test tubes and flasks.

A preferred cis-diol specific affinity medium is a boronate affinity medium. The boronate affinity medium comprises boronic acid groups of the formula -RB(OH)₂ and/or of the formula -RB(OH)₃⁻, depending on the pH, wherein the boronic acid groups are coupled to the matrix of the affinity medium via R by covalent or non-covalent interactions. Preferably, R comprises an optionally substituted aryl or optionally substituted alkyl group, wherein the substituents may be any substituents as long as they do not obstruct the binding of catecholamines to the boronic acid groups. More preferably, the boronate affinity medium comprises amino boronic acid groups wherein R comprises an optionally substituted aryl or alkyl group substituted with a -NH- group which links the boronic acid group to the matrix. A preferred boronate affinity medium comprises m-aminophenyl boronic acid groups of the following formulas:

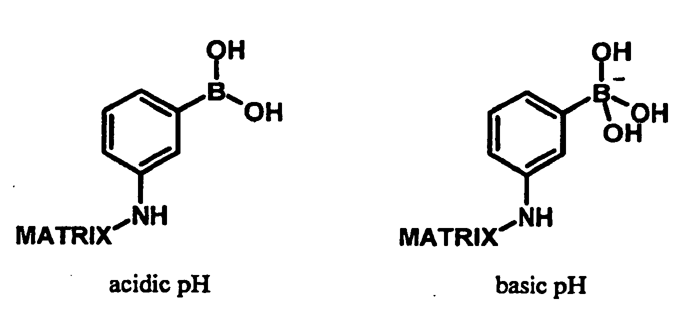

Another example of a cis-diol specific affinity medium is alumina.

According to the present invention, the term "binding" refers to the covalent or non-covalent attachment of one entity to another entity such as catecholamines to the cis-diol specific groups of a cis-diol specific affinity medium. The term "binding" includes specific and non-specific binding. Binding may be achieved, for example, by covalent bonds or ionic, polar or nonpolar interactions. Binding may require special conditions to be met, including pH value, ionic strength, temperature, solvents and additives. Preferably, the binding is strong enough to withstand one or more rinsing steps.

If the cis-diol specific groups are boronic acid groups, binding to a catecholamine is achieved by a covalent bond between the cis-diol structure of the catechol moiety of the catecholamine and the boronic acid group, as shown in the following formula:

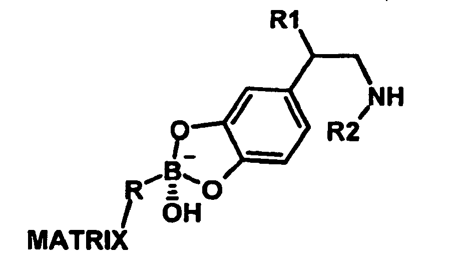

The term "releasing of catecholamines bound to a cis-diol specific affinity medium" refers to disrupting the binding of the catecholamines to the cis-diol specific affinity medium. This may be achieved by changing one or more of the conditions required for binding of the catecholamines to the cis-diol specific affinity medium and/or by addition of a releasing agent. Most preferably, catecholamines are released from a cis-diol specific affinity medium by adjusting the pH to acidic conditions.

According to the present invention the term "blocking the binding of catecholamines to a cis-diol specific affinity medium" refers to the inhibition of the binding of catecholamines, not bound to said cis-diol specific affinity medium, to said cis-diol specific affinity medium. This may be achieved by the addition of a substance (blocking substance) which blocks the binding of catecholamines to the cis-diol specific affinity medium under conditions which otherwise would be appropriate for binding. The blocking substance may be a single compound or a composition, i.e. a mixture, of compounds, including one or more blocking compounds. Furthermore, the blocking substance may block the binding of one particular catecholamine, a particular group of catecholamines or all catecholamines.
Preferably, the blocking does not alter the chemical structure of the catecholamines. "Competitive binding" in the context of the present invention refers to binding of the blocking substance to the cis-diol specific affinity medium, thus inhibiting its interaction with catecholamines. Preferably, the blocking substance is added in excess amounts.

According to the present invention, the term "enzymatic reaction" refers to any reaction that is catalyzed by an enzyme. An enzyme is any protein, polypeptide or derivative thereof, such as those containing enzymes, capable of catalyzing a chemical reaction.

According to the present invention, the term "modification of catecholamines" relates to any modification of catecholamines as found in a sample and includes, in particular, modifications by acylation and/or O-alkylation.

As used herein, the term "O-alkylation" of a substance refers to a reaction, wherein an alkyl group is covalently bound to an oxygen atom of said substance. The alkyl group may be any suitable alkyl group, including optionally substituted, branched and unbranched, saturated and unsaturated alkyl groups, optionally comprising heteroatoms. Preferably, the alkyl group is a methyl group. "O-methylation" of a substance refers to a reaction, wherein a methyl group is covalently bound to an oxygen atom of said substance.

According to the present invention, the term "acylation" of a substance refers to a reaction, wherein an acyl group is covalently bound to said substance. An acyl group is any group of the formula -C(O)R, wherein R is an alkyl or aryl group, including optionally substituted, branched and unbranched, saturated and unsaturated alkyl or aryl groups, optionally comprising heteroatoms. Acyl groups for covalent binding to catecholamines may contain a label such as a radioisotope. An example of an acylation reagent suitable for the use in the present invention is Bolton-Hunter reagent:

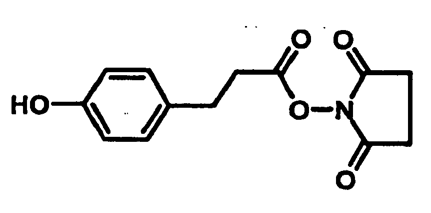

As used herein, the term "immunoassay" refers to a test system for detection and/or quantification of a compound which includes binding said compound to an antibody specific thereto and detecting the compound-antibody complex. An immunoassay may provide information on the presence or absence of the compound and/or on the relative or absolute concentration or amount of the compound. The detection and/or quantification may be accomplished using a detectable label such as enzymes, fluorescent labels, luminescent labels and radioactive labels. Examples of suitable enzymes are peroxidases such as horseradish peroxidase, β-galactosidase and phosphatases such as alkaline phosphatase.

In one example of an immunoassay, a defined amount of a competitor for the compound of interest is added together with an antibody specific for the competitor/compound of interest. The competitor may be the same compound as the compound of interest, but may differ from the compound of interest in that it bears a detectable label such as described above, e.g. a radioisotope such as ¹²⁵I, or in that it is immobilized on a support. The antibody complexes formed may then be bound by a secondary antibody immobilized on a support, if the competitor is detectably labeled, or by a secondary antibody coupled to a detectable label, e.g. an enzyme such as horseradish peroxidase, if the competitor is immobilized on a support. A suitable support includes precipitation agents, e.g. beads such as sepharose beads, and the walls of a reaction vessel.

### DESCRIPTION OF A PREFERRED EMBODIMENT

In the following, a preferred embodiment of the method of the present invention is described, wherein catecholamines contained in a biological sample are analyzed.

In the first step, the sample is added into a reaction vessel comprising a boronate affinity medium attached to it. At neutral to basic conditions, e.g. a pH of about 8, the catecholamines contained in the sample bind to the boronic acid groups of the boronate affinity medium. The remainder of the sample is removed, e.g. by decanting or suction, and the reaction vessel rinsed.

In the second step, an acylation reagent is added, e.g. a solution containing Bolton-Hunter reagent, acylating the bound catecholamines. After completion of the acylation reaction the solution is removed and the reaction vessel rinsed.

In the third step, the bound catecholamines are released from the boronate affinity medium by adjusting the pH to acidic conditions, e.g. about 2. This is achieved by addition of an acid, e.g. hydrochloric acid. Simultaneously, a blocking substance, e.g. fructose or sorbitol, is added in a final concentration depending on the binding capacity of the boronate affinity gel and the affinity of the blocking substance to the boronate affinity gel.

In the fourth step, an enzyme solution for O-methylation of the released catecholamines is added, e.g. comprising S-adenosylmethionine and the enzyme COMT, which transfers a methyl group to the 3-hydroxyl group of the catechol moiety of the catecholamines. Simultaneously, the pH of the solution containing the catecholamines is adjusted to neutral to basic conditions, e.g. a pH of about 7.5. Rebinding of the catecholamines to the boronate affinity medium under these pH conditions is blocked by the blocking substance added in the previous step.

In the fifth step, the presence and/or concentration of the modified catecholamines contained in the reaction vessel is analyzed using an immunoassay, e.g. an enzyme or radio immunoassay, as described above.

As obvious from the disclosure of the present invention, a method is provided that allows to perform the extraction and modification of catecholamines all in one reaction vessel, resulting in the final catecholamine derivative useful for enzyme or radio immunoassays. Thus, the catecholamines prepared according to the method of the invention are suitable for analysis via an EIA using a standard automated analyzer. Thereby, multiple EIAs analyzing different catecholamines may be performed using the modified catecholamines prepared by only one extraction procedure.

Alternatively, by using the appropriate reaction vessel as described herein it is possible to perform the EIA in the same reaction vessel which was used for the extraction and modification of the catecholamines.

A suitable reaction vessel comprises a cis-diol specific affinity medium and a catecholamine or an antibody specific for a catecholamine specific antibody immobilized to the reaction vessel. One example of such a reaction vessel is a pin plate which consists of a plate with one or more wells and a top cover comprising one or more pins which project into the recesses of the wells. For the use of a pin plate in the method of the present invention, a cis-diol specific affinity medium may be attached to the bottom and/or wall of one or more wells of the pin plate and catecholamines or antibodies specific for a catecholamine specific antibody may be attached to one or more pins of the pin plate. Pin plates are know in the art and a pin plate may be produced by a conventional process.

In the following, an example of performing the method of the present invention according to the preferred embodiments is described.

### EXAMPLE 1

### Extraction of epinephrine from urine and its quantitative determination by EIA in one reaction vessel

10 µl urine and 25 µl assay buffer (1 M sodium hydrogencarbonate) are added into a reaction vessel coated with (i) a boronate affinity medium comprising m-aminophenyl boronic acid groups and (ii) 3-O-methyl-norepinephrine attached to the reaction vessel via an acyl group bound to the nitrogen atom. After 30 min incubation at room temperature (RT) on a shaker, the reaction vessel is washed 3 times with 300 µl washing buffer (10 mM Tris/HCl pH 7.4, 150 mM sodium chloride, 0.1 % (w/v) Tween 20). 100 µl assay buffer and 25 µl acylation reagent (0.8 M succinic acid anhydride in dimethyl sulfoxide/dimethylformamide, v/v, 1/1) are added and the reaction is incubated for 15 min at RT on a shaker. After removal of the fluid the reaction vessel is again washed 3 times with 300 µl washing buffer. 150 µl release buffer (25 mM hydrochloric acid, 20 g/l sorbitol, 4 mg/l phenol red) are added and after 5 min incubation at RT on a shaker 50 µl enzyme solution (COMT, 0,125 g/l S-adenosyl-L-methionine, 350 mM Tris/HCl pH 9.1, 5 mM MgCl₂, 0,125 g/l dithiotreitol) are added. The reaction is incubated for 15 min at RT on a shaker and 50 µl antiserum from rabbit immunized against N-acyl metanephrine (N-acylated and 3-O-methylated epinephrine) are added. After incubation for 60 min at RT on a shaker the fluid is removed and the reaction vessel is washed 3 times with 300 µl washing buffer. 100 µl enzyme conjugate (goat anti-rabbit IgG monoclonal antibody conjugated to a peroxidase) are added and incubated for 30 min at RT on a shaker. After removal of the fluid the reaction vessel is washed 3 times with 300 µl washing buffer and 100 µl substrate (3,3',5,5'-tetramethylbenzidine (Blue Star TMB)) are added. The reaction is incubated for 30 min at RT on a shaker and thereafter stopped by adding of 100 µl stop solution (0.25 M H₂SO₄). The product of the enzymatic reaction is detected at 450 nm. The concentration of epinephrine in the sample can be determined by comparing the detected OD with an established calibration curve, whereby the OD increases with decreasing epinephrine concentration in the sample.

### EXAMPLE 2

Extraction of epinephrine from urine and its quantitative determination by EIA using a standard automated EIA analyzer

The extraction and derivatization of the catecholamines is performed as in example 1, except that a reaction vessel comprising only the boronate affinity gel is used. After the incubation of the enzymatic reaction using COMT, the solution containing the modified catecholamine is transferred into a reaction vessel suitable for a standard automated EIA analyzer and the EIA is performed in an automated manner using such an analyzer.

### EXAMPLE 3

### Extraction of epinephrine from urine and its quantitative determination by RIA

15µl urine and 25 µl assay buffer (1 M sodium hydrogencarbonate) are added into a reaction vessel coated with a boronate affinity medium comprising m-aminophenyl boronic acid groups. After 30 min incubation at room temperature (RT) on a shaker, the reaction vessel is washed 3 times with 300 µl washing buffer (150 mM sodium chloride). 100 µl assay buffer and 25 µl acylation reagent (0.8 M succinic acid anhydride in dimethyl sulfoxide/ dimethylformamide, v/v, 1/1) are added and the reaction is incubated for 15 min at RT on a shaker. After removal of the fluid the reaction vessel is again washed 3 times with 300 µl washing buffer. 150 µl release buffer (25 mM hydrochloric acid, 20 g/l sorbitol, 4 mg/l phenol red) are added and after 5 min incubation at RT on a shaker 50 µl enzyme solution (COMT, 0,125 g/l S-adenosyl-L-methionine, 350 mM Tris/HCl pH 9.1, 5 mM MgC1₂, 0,125 g/l dithiotreitol) are added. The reaction is incubated for 30 min at RT on a shaker and 50 µl antiserum from rabbit immunized against N-acyl metanephrine (N-acylated and 3-O-methylated epinephrine) and radioactively labeled N-acyl metanephrine (<100 kBq) are added. After further incubation for 1 h, 1 ml precipitating reagent (goat anti rabbit antibodies in 5% PEG 6000 solution) is added and incubated for 15 min at 2 - 8 °C. The reaction solution is then centrifuged for 15 min. at 3000 x g and the supernatant is carefully decanted or aspirated (except totals). The radioactivity retained in the reaction vessel is then counted for 1 min in a gamma counter.

## Claims

1. A method for analyzing at least one catecholamine comprising the steps of:
(a) binding the at least one catecholamine to a cis-diol specific affinity medium;
(b) adding a substance that blocks the binding of catecholamines to the cis-diol specific affinity medium by competitive binding to the cis-diol specific affinity medium;
(c) releasing the at least one catecholamine bound to the cis-diol specific affinity medium from the cis-diol specific affinity medium; and
(d) subjecting the at least one catecholamine to at least one reaction which requires non-acidic conditions;
wherein step (b) and step (c) are performed simultaneously, or step (b) is performed prior to or following step (c), and wherein step (a), step (b), step (c) and step (d) are all done within the same reaction vessel.

2. The method of claim 1 wherein the at least one catecholamine comprises a naturally occurring and/or a synthetic catecholamine.

3. The method of claim 2 wherein the naturally occurring catecholamine is selected from the group consisting of adrenalin, noradrenaline, dopamine, epinephrine, norepinephrine and mixtures thereof.

4. The method of any one of claims 1 to 3 wherein the at least one catecholamine is contained in a biological sample or a fraction thereof.

5. The method of claim 4 wherein the biological sample is a urine sample or a plasma sample.

6. The method of any one of claims 1 to 5 wherein the cis-diol specific affinity medium is a boronate affinity medium.

7. The method of claim 6 wherein the boronate affinity medium comprises amino boronic acid groups.

8. The method of claim 6 or 7 wherein the boronate affinity medium comprises m-aminophenyl boronic acid groups.

9. The method of any one of claims 1 to 8 wherein step (a) takes place at a neutral to basic pH.

10. The method of any one of claims 1 to 9 wherein the substance added in step (b) contains at least one cis-diol structure.

11. The method of claim 10 wherein the substance is selected from the group consisting of 1,2-dihydroxyalkanes, polyphenols, 2-hydroxy acids, ascorbic acid, carbohydrates, derivatives thereof and mixtures thereof.

12. The method of claim 11 wherein the 1,2-dihydroxyalkane is ethanediol, 1,2-propanediol, or an alkanediol comprising vicinal hydroxyl groups.

13. The method of claim 11 wherein the polyphenol is a catechol, anthocyane, gallic acid, or tannin.

14. The method of claim 11 wherein the 2-hydroxyacid is lactic acid, tartaric acid, or citric acid.

15. The method of claim 11 wherein the carbohydrate is a monosaccharide, a disaccharide, an oligosaccharide or a polysaccharide.

16. The method of claim 11 wherein the carbohydrate derivative is selected from the group consisting of nucleosides, nucleotides, oligo- and polynucleotides, and glycoproteins.

17. The method of claim 16 wherein the oligo- or polynucleotide is RNA.

18. The method of claim 11 wherein the substance is sorbitol or fructose.

19. The method of any one of claims 1 to 18 wherein the at least one catecholamine is released from the cis-diol specific affinity medium by addition of an acid.

20. The method of claim 19 wherein the acid is selected from the group consisting of hydrochloric acid, perchloric acid, and mixtures thereof.

21. The method of any one of claims 1 to 20 wherein in step (c) the pH is 3 or lower.

22. The method of any one of claims 1 to 21 wherein the concentration of the substance added in step (b) is sufficient to block the binding of at least 95%, preferably at least 99%, of the at least one catecholamine released from the cis-diol specific affinity medium to the cis-diol specific affinity medium.

23. The method of any one of claims 1 to 22 wherein the substance added in step (b) is added to a final concentration that equals or exceeds the binding capacity of the used cis-diol specific affinity medium.

24. The method of any one of claims 1 to 23 wherein in step (d) the at least one reaction is an enzymatic reaction.

25. The method of any one of claim 1 to 24 wherein in step (d) the at least one reaction is an O-alkylation, preferably an O-methylation.

26. The method of claim 25 wherein the O-methylation is effected by means of the enzyme catechol-3-O-methyl-transferase.

27. The method of any one of claims 1 to 26 wherein in step (d) the non-acidic conditions include a pH of 6 or higher.

28. The method of any one of claims 1 to 27 further comprising the step of acylating the at least one catecholamine.

29. The method of claim 28 wherein the at least one catecholamine is acylated while bound to the cis-diol specific affinity medium.

30. The method of any one of claims 1 to 29 further comprising the step of detecting and/or quantifying the at least one catecholamine, preferably using an antibody specific for a catecholamine or modified catecholamine.

31. The method of claim 30 wherein the detection and/or quantification comprises an enzyme or radio immunoassay.

32. The method of claim 30 or 31 wherein step (a), step (b), step (c), step (d) and the step of detection and/or quantification of the at least one catecholamine are done within the same reaction vessel.

## Patentansprüche

1. Verfahren zur Analyse mindestens eines Catecholamins, das die Schritte umfasst:
(a) Binden des mindestens einen Catecholamins an ein cis-diol-spezifisches Affinitätsmedium,
(b) Zugeben einer Substanz, die die Bindung von Catecholaminen an das cis-diol-spezifische Affinitätsmedium durch kompetitive Bindung an das cis-diol-spezifische Affinitätsmedium blockiert,
(c) Freisetzen des mindestens einen an das cis-diol-spezifische Affinitätsmedium gebundenen Catecholamins von dem cis-diol-spezifischen Affinitätsmedium und
(d) Unterwerfen des mindestens einen Catecholamins mindestens einer Reaktion, die nicht-saure Bedingungen erfordert,
wobei Schritt (b) und (c) gleichzeitig durchgeführt werden oder Schritt (b) vor oder nach Schritt (c) durchgeführt wird und wobei Schritt (a), Schritt (b), Schritt (c) und Schritt (d) alle innerhalb des gleichen Reaktionsgefäßes erfolgen.

2. Verfahren nach Anspruch 1, wobei das mindestens eine Catecholamin ein natürlich auftretendes und/oder ein synthetisches Catecholamin umfasst.

3. Verfahren nach Anspruch 2, wobei das natürlich auftretende Catecholamin ausgewählt ist aus der Gruppe, bestehend aus Adrenalin, Noradrenalin, Dopamin, Epinephrin, Norepinephrin und Gemischen davon.

4. Verfahren nach einem der Ansprüche 1 bis 3, wobei das mindestens eine Catecholamin in einer biologischen Probe oder einer Fraktion davon enthalten ist.

5. Verfahren nach Anspruch 4, wobei die biologische Probe eine Urinprobe oder eine Plasmaprobe ist.

6. Verfahren nach einem der Ansprüche 1 bis 5, wobei das cis-diol-spezifische Affinitätsmedium ein Boronat-Affinitätsmedium ist.

7. Verfahren nach Anspruch 6, wobei das Boronat-Affinitätsmedium Aminoboronsäuregruppen umfasst.

8. Verfahren nach Anspruch 6 oder 7, wobei das Boronat-Affinitätsmedium m-Aminophenylboronsäuregruppen umfasst.

9. Verfahren nach einem der Ansprüche 1 bis 8, wobei Schritt (a) bei einem neutralen bis basischen pH-Wert erfolgt.

10. Verfahren nach einem der Ansprüche 1 bis 9, wobei die in Schritt (b) hinzugefügte Substanz mindestens eine cis-diol-Struktur enthält.

11. Verfahren nach Anspruch 10, wobei die Substanz ausgewählt ist aus der Gruppe, bestehend aus 1,2-Dihydroxyalkanen, Polyphenolen, 2-Hydroxysäuren, Ascorbinsäure, Kohlenhydraten, Derivaten davon und Gemischen davon.

12. Verfahren nach Anspruch 11, wobei das 1,2-Dihydroxyalkan Ethandiol, 1,2-Propandiol oder ein Alkandiol, das benachbarte Hydroxylgruppen umfasst, ist.

13. Verfahren nach Anspruch 11, wobei das Polyphenol ein Catechol, Anthocyan, Gallensäure oder Tannin ist.

14. Verfahren nach Anspruch 11, wobei die 2-Hydroxysäure Milchsäure, Weinsäure oder Zitronensäure ist.

15. Verfahren nach Anspruch 11, wobei das Kohlenhydrat ein Monosaccharid, ein Disaccharid, ein Oligosaccharid oder ein Polysaccharid ist.

16. Verfahren nach Anspruch 11, wobei das Kohlenhydratderivat ausgewählt ist aus der Gruppe, bestehend aus Nukleosiden, Nukleotiden, Oligo- und Polynukleotiden und Glycoproteinen.

17. Verfahren nach Anspruch 16, wobei das Oligo- oder Polynukleotid RNA ist.

18. Verfahren nach Anspruch 11, wobei die Substanz Sorbitol oder Fructose ist.

19. Verfahren nach einem der Ansprüche 1 bis 18, wobei das mindestens eine Catecholamin von dem cis-diol-spezifischen Affinitätsmedium durch Zugabe einer Säure freigesetzt wird.

20. Verfahren nach Anspruch 19, wobei die Säure ausgewählt ist aus der Gruppe, bestehend aus Chlorwasserstoffsäure, Perchlorsäure und Gemischen davon.

21. Verfahren nach einem der Ansprüche 1 bis 20, wobei in Schritt (c) der pH-Wert 3 oder weniger beträgt.

22. Verfahren nach einem der Ansprüche 1 bis 21, wobei die Konzentration der in Schritt (b) zugefügten Substanz ausreichend ist, um die Bindung von mindestens 95%, vorzugsweise mindestens 99% des mindestens einen Catecholamins, das von dem cis-diol-spezifischen Affinitätsmedium freigesetzt wurde, an das cis-diol-spezifische Affinitätsmedium zu blockieren.

23. Verfahren nach einem der Ansprüche 1 bis 22, wobei die in Schritt (b) zugefügte Substanz in einer Endkonzentration hinzugefügt wird, die der Bindekapazität des verwendeten cis-diol-spezifischen Affinitätsmediums entspricht oder diese übersteigt.

24. Verfahren nach einem der Ansprüche 1 bis 23, wobei in Schritt (d) die mindestens eine Reaktion eine enzymatische Reaktion ist.

25. Verfahren nach einem der Ansprüche 1 bis 24, wobei in Schritt (d) die mindestens eine Reaktion eine O-Alkylierung, vorzugsweise eine O-Methylierung ist.

26. Verfahren nach Anspruch 25, wobei die O-Methylierung mittels des Enzyms Catechol-3-O-Methyltransferase erfolgt.

27. Verfahren nach einem der Ansprüche 1 bis 26, wobei in Schritt (d) die nicht-sauren Bedingungen einen pH-Wert von 6 oder mehr beinhalten.

28. Verfahren nach einem der Ansprüche 1 bis 27, das ferner den Schritt einer Acylierung des mindestens einen Catecholamins umfasst.

29. Verfahren nach Anspruch 28, wobei das mindestens eine Catecholamin acyliert wird, während es an das cis-diol-spezifische Affinitätsmedium gebunden ist.

30. Verfahren nach einem der Ansprüche 1 bis 29, das ferner den Schritt eines Nachweises und/oder einer Quantifizierung des mindestens einen Catecholamins, vorzugsweise durch Verwendung eines Antikörpers, der für ein Catecholamin oder ein modifiziertes Catecholamin spezifisch ist, umfasst.

31. Verfahren nach Anspruch 30, wobei der Nachweis und/oder die Quantifizierung einen Enzym- oder Radioimmunassay umfasst.

32. Verfahren nach Anspruch 30 oder 31, wobei Schritt (a), Schritt (b), Schritt (c), Schritt (d) und der Schritt eines Nachweises und/oder einer Quantifizierung des mindestens einen Catecholamins innerhalb des gleichen Reaktionsgefäßes erfolgen.

## Revendications

1. Procédé pour l'analyse d'au moins une catécholamine, comprenant les étapes consistant à:
(a) lier la au moins une catécholamine à un milieu d'affinité spécifique pour un cis-diol;
(b) ajouter une substance qui bloque la liaison des catécholamines au milieu d'affinité spécifique pour un cis-diol par liaison compétitive au milieu d'affinité spécifique pour un cis-diol;
(c) libérer la au moins une catécholamine liée au milieu d'affinité spécifique pour un cis-diol du milieu d'affinité spécifique pour un cis-diol; et
(d) soumettre la au moins une catécholamine à au moins une réaction qui nécessite des conditions non-acides;
tandis que l'étape (b) et l'étape (c) sont mises en oeuvre simultanément, ou l'étape (b) est mise en oeuvre avant ou après l'étape (c), et tandis que l'étape (a), l'étape (b), l'étape (c) et l'étape (d) sont toutes effectuées dans le même récipient de réaction.

2. Procédé selon la revendication 1, dans lequel la au moins une catécholamine comprend une catécholamine naturelle et/ou synthétique.

3. Procédé selon la revendication 2, dans lequel la catécholamine naturelle est choisie dans le groupe consistant en adrénaline, noradrénaline, dopamine, épinéphrine, norépinéphrine et leurs mélanges.

4. Procédé selon l'une quelconque des revendications 1 à 3, dans lequel la au moins une catécholamine est contenue dans un échantillon biologique ou une fraction de celui-ci.

5. Procédé selon la revendication 4, dans lequel l'échantillon biologique est un échantillon d'urine ou un échantillon de plasma.

6. Procédé selon l'une quelconque des revendications 1 à 5, dans lequel le milieu d'affinité spécifique pour un cis-diol est un milieu d'affinité au borate.

7. Procédé selon la revendication 6, dans lequel le milieu d'affinité au borate comprend des groupes acide amino borique.

8. Procédé selon la revendication 6 ou 7, dans lequel le milieu d'affinité au borate comprend des groupes acide m-aminophényl borique.

9. Procédé selon l'une quelconque des revendications 1 à 8, dans lequel l'étape (a) prend place à un pH de neutre à basique.

10. Procédé selon l'une quelconque des revendications 1 à 9, dans lequel la substance ajoutée dans l'étape (b) contient au moins une structure cis-diol.

11. Procédé selon la revendication 10, dans lequel la substance est choisie dans le groupe consistant en 1,2-dihydroxyalcanes, polyphénols, 2-hydroxy acides, acide ascorbique, hydrates de carbone, dérivés de ceux-ci et mélanges de ceux-ci.

12. Procédé selon la revendication 11, dans lequel le 1,2-dihydroxyalcane est l'éthanediol, le 1,2-propanediol, ou un alcanediol comprenant des groupes hydroxyle vicinaux.

13. Procédé selon la revendication 11, dans lequel le polyphénol est un catéchol, anthocyane, acide gallique, ou tanin.

14. Procédé selon la revendication 11, dans lequel le 2-hydroxyacide est l'acide lactique, l'acide tartrique, ou l'acide citrique.

15. Procédé selon la revendication 11, dans lequel l'hydrate de carbone est un monosaccharide, un disaccharide, un oligosaccharide ou un polysaccharide.

16. Procédé selon la revendication 11, dans lequel le dérivé d'hydrate de carbone est choisi dans le groupe consistant en nucléosides, nucléotides, oligo- et polynucléotides, et glycoprotéines.

17. Procédé selon la revendication 16, dans lequel l'oligo- ou polynucléotide est l'ARN.

18. Procédé selon la revendication 11, dans lequel la substance est le sorbitol ou le fructose.

19. Procédé selon l'une quelconque des revendications 1 à 18, dans lequel la au moins une catécholamine est libérée du milieu d'affinité spécifique pour un cis-diol par addition d'un acide.

20. Procédé selon la revendication 19, dans lequel l'acide est choisi dans le groupe consistant en acide chlorhydrique, acide perchlorique, et des mélanges de ceux-ci.

21. Procédé selon l'une quelconque des revendications 1 à 20, dans lequel dans l'étape (c) le pH est de 3 ou moins.

22. Procédé selon l'une quelconque des revendications 1 à 21, dans lequel la concentration de la substance ajoutée dans l'étape (b) est suffisante pour bloquer la liaison d'au moins 95%, de préférence d'au moins 99%, de la au moins une catécholamine libérée à partir du milieu d'affinité spécifique pour un cis-diol du milieu d'affinité spécifique pour un cis-diol.

23. Procédé selon l'une quelconque des revendications 1 à 22, dans lequel la substance ajoutée dans l'étape (b) est ajoutée à une concentration finale qui est égale ou supérieure à la capacité de liaison du milieu d'affinité spécifique pour un cis-diol utilisé.

24. Procédé selon l'une quelconque des revendications 1 à 23, dans lequel dans l'étape (d) la au moins une réaction est une réaction enzymatique.

25. Procédé selon l'une quelconque des revendications 1 à 24, dans lequel dans l'étape (d) la au moins une réaction est une O-alkylation, de préférence une O-méthylation.

26. Procédé selon la revendication 25, dans lequel la O-méthylation est effectuée au moyen de l'enzyme catéchol-3-O-méthyl-transférase.

27. Procédé selon l'une quelconque des revendications 1 à 26, dans lequel dans l'étape (d) les conditions non-acides comprennent un pH de 6 ou plus.

28. Procédé selon l'une quelconque des revendications 1 à 27, comprenant en outre l'étape d'acylation de la au moins une catécholamine.

29. Procédé selon la revendication 28, dans lequel la au moins une catécholamine est acylée tandis qu'elle est liée au milieu d'affinité spécifique pour un cis-diol.

30. Procédé selon l'une quelconque des revendications 1 à 29, comprenant en outre l'étape de détection et/ ou de quantification d'au moins une catécholamine, de préférence en utilisant un anticorps spécifique pour une catécholamine ou une catécholamine modifiée.

31. Procédé selon la revendication 30, dans lequel la détection et/ou la quantification comprend un dosage par une technique immunoenzymatique ou radio-immunologique.

32. Procédé selon la revendication 30 ou 31, dans lequel l'étape (a), l'étape (b), l'étape (c), l'étape (d) et l'étape de détection et/ou de quantification de la au moins une catécholamine sont effectuées dans le même récipient de réaction.
